# EUROPEAN PATENT APPLICATION

(11) **EP 3 711 798 A1**
(43) Date of publication of application: **23.09.2020**
(21) Application number: 19164420.2
(22) Date of filing: 21.03.2019
(51) Int. Cl.: A61M 11/04, A61M 15/06, A24F 47/00

(54) **AEROSOL DELIVERY SYSTEM**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

An aerosol delivery system has a fluid-transfer article with a first region for holding an aerosol precursor and a second region to which the aerosol precursor is transferred from the first region. The second region includes an activation surface which interacts thermally with a heater of the aerosol delivery system to form an aerosol from said aerosol precursor. The fluid-transfer article includes at least one heat activatable valve which controls transfer of aerosol precursor from the first region. The or each valve may be between the first and second regions, or may form the second region so that a surface of the valve or valves forms the activation surface. The or each valve may limit or prevent escape of aerosol precursor from the fluid-transfer article, when the heater is not active, but allow the aerosol precursor to reach the activation surface when the aerosol delivery system is in use.

## Description

### Field of the Invention

The present invention relates to an aerosol delivery system, an aerosol-generation apparatus and a fluid-transfer article for an aerosol delivery system. Thus, the present invention preferably relates to an aerosol delivery system including a heater configured to heat an aerosol precursor to generate an aerosolised composition for inhalation by a user, and to an aerosol-generation apparatus and a fluid-transfer article therefor.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette® Inhalator. These generally have the appearance of a plastic cigarette and are used by people who crave the behaviour associated with consumption of combustible tobacco - the so- called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, *inter alia,* as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat.

The present invention has been devised in light of the above considerations.

### Summary of the Invention

At its most general, the present invention proposes that a fluid-transfer article has a heat activatable valve, or possibly a plurality of such valves, which controls transfer of aerosol precursor to an activation surface of the article. In use of the fluid-transfer article, the activation surface will normally be disposed at an end of the article configured for thermal interaction with a heating surface of a heater of an aerosol-generation apparatus.

In such an arrangement, the valve may reduce or eliminate escape of aerosol precursor from the article when the article is not being used, and the heater is not activated, but permit aerosol precursor to reach the activation surface to be heated when the article is in use.

Thus, according to a first aspect of the present invention, there may be provided a fluid-transfer article comprising a first region for holding an aerosol precursor and for transferring said aerosol precursor to an activation surface of a second region of said article, said activation surface being disposed at an end of said article configured for thermal interaction with a heating surface of a heater of an aerosol-generation apparatus; the article further including at least one heat activatable valve for controlling the transfer of said aerosol precursor from said first region to said activation surface.

Optionally, the or each heat activatable valve is between the first region and the second region. In such an arrangement, the valve or valves control the transfer of the aerosol precursor from the first region to the second region. The second region may then be arranged to transfer aerosol precursor from the or each valve to the activation surface. To do this, the second region may be formed from a wicking material, preferably a polymeric wicking material.

In a possible arrangement of the present invention said second region may comprise at least one discontinuity in said activation surface to form a corresponding at least one channel between said activation surface and said heating surface, the or each said channel being configured for providing an air-flow pathway across said activation surface as such at least one channel is provided, the activation surface may preferably include at least one angled surface portion and being configured said that, when said fluid transfer article is arranged with respect to said heating surface for thermal interaction therebetween the or each angled surface portion forms an acute intersection angle with said heating surface, the or each said channel being at least partially defined by a said angled surface portion in the form of a wall of said channel. It is then possible that the or each said channel is a least partially defined by a pair of said angled surface portions, said pair of angled surface portions opposing one another across said channel to form opposite walls of said channel.

Alternatively, where the second region comprises at least one discontinuity in the activation surface to form a corresponding at least one channel, the activation surface may include at least one arcuate surface portion and being configured such that, when the fluid transfer article is arranged with respect to said heating surface for thermal interaction therebetween, the or each arcuate surface portion opposes said heating surface and is concave towards said heating surface, said arcuate surface portion defining at least part of an internal surface of said channel.

In another option within the present invention, the heat activatable valve or valves may form the second region of the fluid-transfer article and a surface of the valve or valves may then be the activation surface. In this case, the valve or valves are then the closest part of the fluid-transfer article to the heating surface of the aerosol-generation apparatus.

In this case, the valve or valves may be separable from the first region of the fluid-transfer article. This allows the first region, which contains aerosol precursor, to be replaced without needing to replace the valve.

In any of the above arrangements, the first region may be formed from wicking material, preferably a polymeric wicking material, and that wicking material may be porous.

In any of the above arrangements, it is preferable that the heat to activate the valve is generated by the heater.

In a second aspect of the present invention, there may be provided an aerosol-generation apparatus comprising a heater which includes a heating surface and a fluid-transfer article as discussed above. When the activation surface is formed from a surface of the valve or valves, or when the activation surface is a surface of the second region of the fluid-transfer article, with the valve or valves between the first and second regions such that activation surface is spaced from the heating surface of the heater, to form an air-flow pathway between the activation surface and the heating surface. The heater may include a heat conduction element forming the heating surface of the heater facing the activation surface.

The present invention may further provide an aerosol delivery system comprising an aerosol-generation apparatus as discussed above and a carrier. The carrier may comprise a housing containing the heater and the fluid-transfer article, the housing having an inlet and an outlet in communication with the air-flow pathway.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Figure 1****.** is a perspective view illustration of a system for aerosol delivery according to one or more embodiments of the present invention;
**Figure 2****.** is a cross-section side view illustration of part of an apparatus of the system for aerosol delivery of Figure 1;
**Figure 3****.** is a cross-section side view illustration of the system and apparatus for aerosol delivery of Figure 1;
**Figure 4****.** is a perspective view illustration of an aerosol carrier for use in the system for aerosol delivery according to one or more embodiments of the present invention;
**Figure 5****.** is a cross-section side view of elements of an aerosol carrier and a part of an apparatus of the system for aerosol delivery according to one or more embodiments of the present invention;
**Figure 6****.** is a cross-section side view of elements of an aerosol carrier and a part of an apparatus of the system for aerosol delivery according to one or more embodiments of the present invention;
**Figure 7****.** is a cross-section side view of elements of an aerosol carrier and a part of an apparatus of the system for aerosol delivery according to one or more embodiments of the present invetion
**Figure 8****.** is a cross-section side view of aerosol carrier according to one or more embodiments of the present invention;
**Figure 9****.** is a perspective cross-section side view of the aerosol carrier of Figure 7, and;
**Figure 10****.** is an exploded perspective view illustration of a kit-of-parts for assembling the system according to one or more embodiments of the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

In general outline, one or more embodiments in accordance with the present invention may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

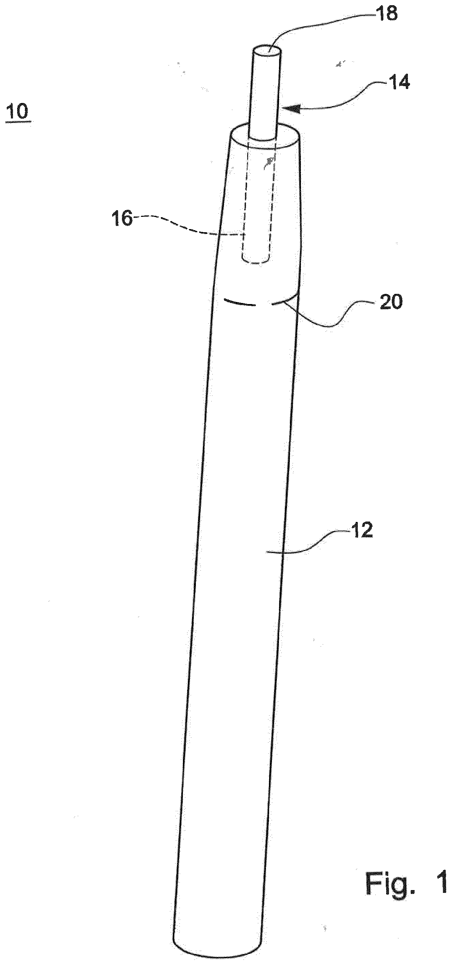

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a first end 16 thereof and a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. A vapour and/or aerosol is produced when a heater (not shown in Figure 1) of the apparatus 12 heats a fluid-transfer article in the aerosol carrier 14 to release a vapour and/or an aerosol, and this can be delivered to the user, when the user sucks or inhales, via a fluid passage in communication with an outlet of the aerosol carrier 14 from the fluid-transfer article t o the second end 18.

The device 12 also comprises air-intake apertures 20 in the housing of the apparatus 12 to provide a passage for air to be drawn into the interior of the apparatus 12 (when the user sucks or inhales) for delivery to the first end 16 of the aerosol carrier 14, so that the air can be drawn across an activation surface of a fluid-transfer article located within a housing of the aerosol carrier cartridge 14 during use. Optionally, these apertures may be perforations in the housing of the apparatus 12.

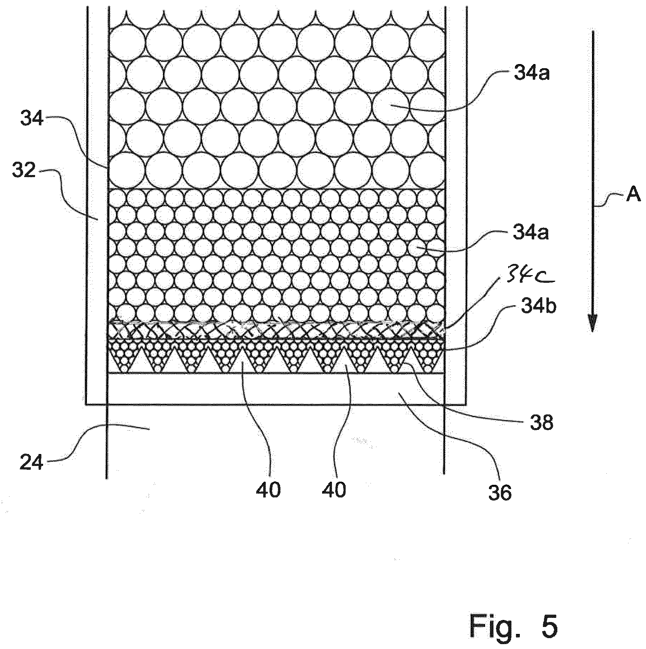

A fluid-transfer article (not shown in Figure 1, but described hereinafter with reference to Figs. 5, 6, 7, 8, 9, and 10) is located within a housing of the aerosol carrier 14. The fluid-transfer article contains an aerosol precursor material, which may include at least one of: nicotine; a nicotine precursor material; a nicotine compound; and one or more flavourings. The fluid-transfer article is located within the housing of the aerosol carrier 14 to allow air drawn into the aerosol carrier 14 at, or proximal, the first end 16 to flow across an activation surface of the fluid-transfer article. As air passes across the activation surface of the fluid-transfer article, an aerosol may be entrained in the air stream from a substrate forming the fluid-transfer article, e.g. via diffusion from the substrate to the air stream and/or via vaporisation of the aerosol precursor material and release from the fluid-transfer article under heating.

The substrate forming the fluid-transfer article 34 comprises a porous material where pores of the porous material hold, contain, carry, or bear the aerosol precursor material. In particular, the porous material of the fluid-transfer article may be a polymeric wicking material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex®. Further optionally, a substrate forming the fluid-transfer article may comprise Polypropylene (PP) or Polyethylene Terephthalate (PET). All such materials may be described as heat resistant polymeric wicking material in the context of the present invention.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

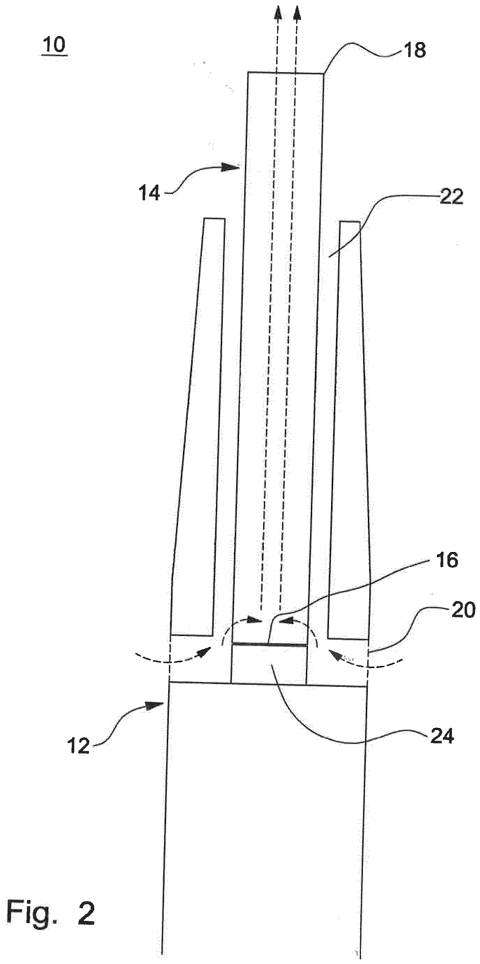

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The apparatus 12 also comprise a heater 24, which opposes an activation surface of the fluid-transfer article (not shown in Figure 2) of the aerosol carrier 14 when an aerosol carrier 14 is located within the receptacle 22.

Air flows into the apparatus 12 (in particular, into a closed end of the receptacle 22) via air-intake apertures 20. From the closed end of the receptacle 22, the air is drawn into the aerosol carrier 14 (under the action of the user inhaling or sucking on the second end 18) and expelled at the second end 18. As the air flows into the aerosol carrier 14, it passes across the activation surface of the fluid-transfer article. Heat from the heater 24, which opposes the activation surface of the fluid-transfer article, causes vaporisation of aerosol precursor material at the activation surface of the fluid-transfer article and an aerosol is created in the air flowing over the activation surface. Thus, through the application of heat in the region of the activation surface of the fluid-transfer article, an aerosol is released, or liberated, from the fluid-transfer article, and is drawn from the material of the aerosol carrier unit by the air flowing across the activation surface and is transported in the air flow to via outlet conduits (not shown in Figure 2) in the housing of the aerosol carrier 14 to the second end 18. The direction of air flow is illustrated by arrows in Figure 2.

To achieve release of the captive aerosol from the fluid-transfer article, the fluid-transfer article of the aerosol carrier 14 is heated by the heater 24. As a user sucks or inhales on second end 18 of the aerosol carrier 14, the aerosol released from the fluid-transfer article and entrained in the air flowing across the activation surface of the fluid-transfer article is drawn through the outlet conduits (not shown) in the housing of the aerosol carrier 14 towards the second end 18 and onwards into the user's mouth.

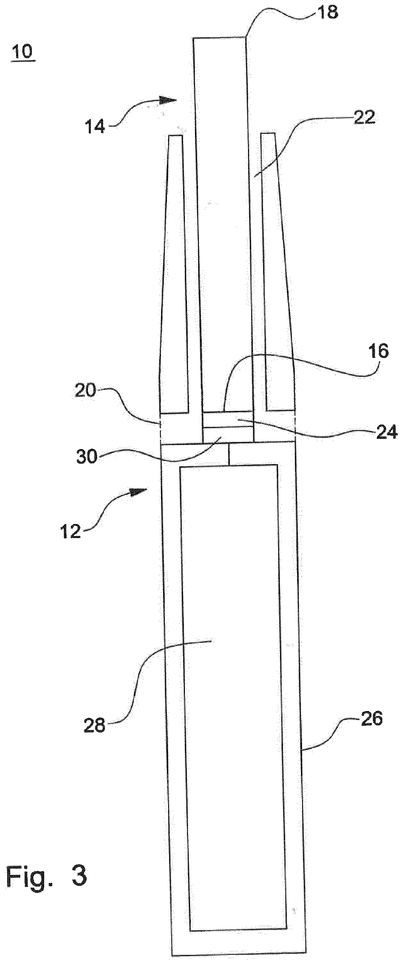

Turning now to Figure 3, a cross-sectional side view of the aerosol delivery system 10 is schematically illustrated showing the features described above in relation to Figures 1 and 2 in more detail. As can be seen, apparatus 12 comprises a housing 26, in which are located the receptacle 22 and heater 24. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater 24.

Responsive to activation of the control circuitry of apparatus 12, the heater 24 heats the fluid-transfer article (not shown in Figure 3) of aerosol carrier 14. This heating process initiates (and, through continued operation, maintains) release of vapour and/or an aerosol from the activation surface of the fluid-transfer article. The vapour and/or aerosol formed as a result of the heating process is entrained into a stream of air being drawn across the activation surface of the fluid-transfer article (as the user sucks or inhales). The stream of air with the entrained vapour and/or aerosol passes through the aerosol carrier 14 via outlet conduits (not shown) and exits the aerosol carrier 14 at second end 18 for delivery to the user. This process is briefly described above in relation to Figure 2, where arrows schematically denote the flow of the air stream into the device 12 and through the aerosol carrier 14, and the flow of the air stream with the entrained vapour and/or aerosol through the aerosol carrier cartridge 14.

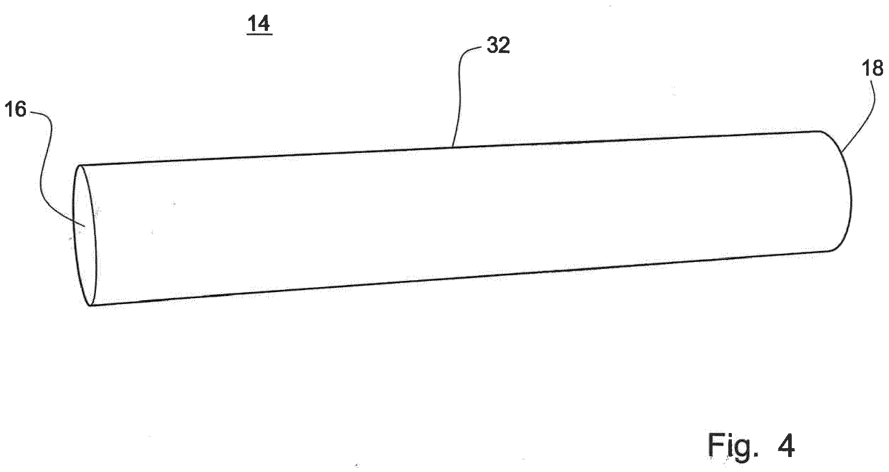

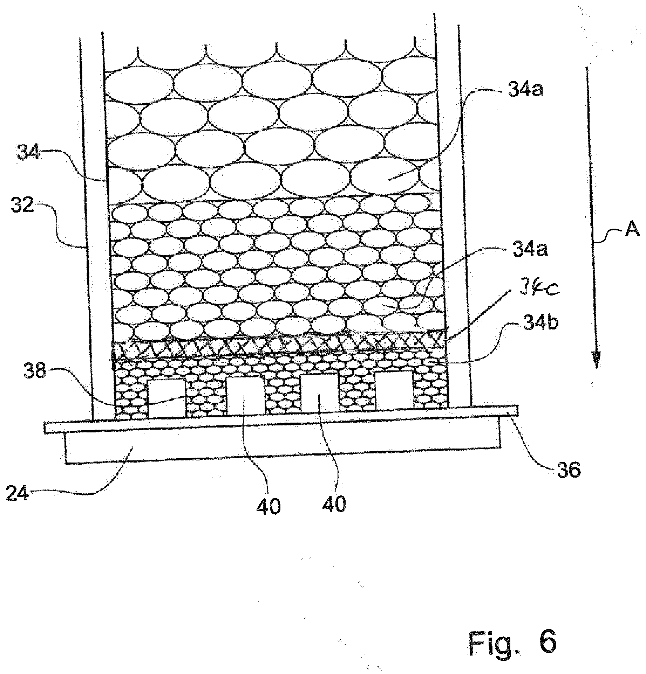

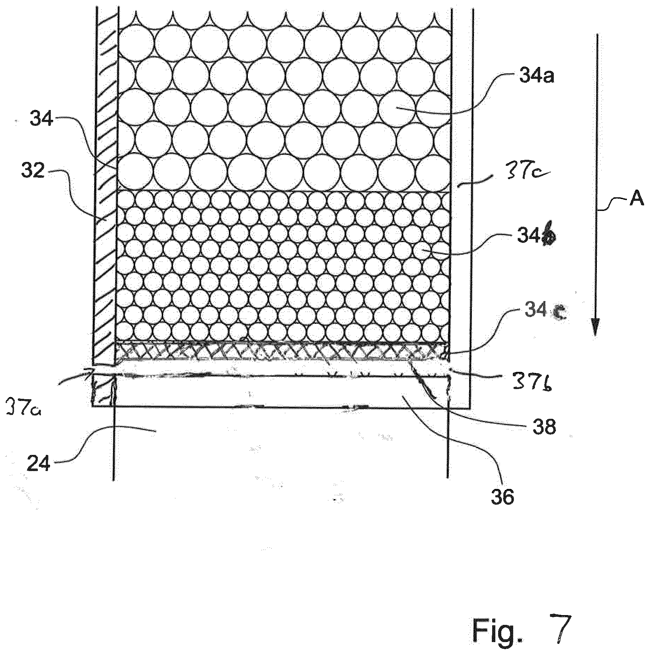

Figures 4 to 7 schematically illustrate the aerosol carrier 14 in more detail (and, in Figures 5 to 7, features within the receptacle in more detail). Figure 4 illustrates an exterior of the aerosol carrier 14, Figure 5 illustrates internal components of the aerosol carrier 14 in an optional configuration, and Fig. 6 illustrates internal components of the aerosol carrier 14 in another optional configuration, and Figure 7 illustrates another embodiment of the internal components of the aerosol carrier 14.

Fig. 4 illustrates the exterior of the aerosol carrier 14, which comprises housing 32 for housing said fluid- transfer article (not shown) and at least one other internal component. The particular housing 32 illustrated in Figure 4 comprises a tubular member, which may be generally cylindrical in form, and which is configured to be received within the receptacle of the apparatus. First end 16 of the aerosol carrier 14 is for location to oppose the heater of the apparatus, and second end 18 (and the region adjacent the second end 18) is configured for insertion into a user's mouth.

Figures 5 to 7 illustrate some optional arrangements corresponding to the internal components of the aerosol carrier 14 and of the heater 24 of apparatus 12.

As described above, the aerosol carrier 14 comprises a fluid-transfer article 34. The aerosol carrier 14 optionally may comprise a conduction element 36 (as shown in Figure 5). In one or more arrangements, the aerosol carrier 14 is located within the receptacle of the apparatus such that the activation surface of the fluid-transfer article opposes the heater of the apparatus and receives heat directly from the heater of the apparatus. In an optional arrangement, such as illustrated in Figure 5 for example, the aerosol carrier 14 comprises a conduction element 36. When aerosol carrier 14 is located within the receptacle of the apparatus such that the activation surface of the fluid-transfer article is located to oppose the heater of the apparatus, the conduction element is disposed between the heater 24 and the activation surface of the fluid-transfer article. Heat may be transferred to the activation surface via conduction through conduction element 36 (i.e. application of heat to the activation surface is indirect).

Further components not shown in Figures. 5 to 7 comprise: an inlet conduit, via which air can be drawn into the aerosol carrier 14; an outlet conduit, via which an air stream entrained with aerosol can be drawn from the aerosol carrier 14; a filter element; and a reservoir for storing aerosol precursor material and for providing the aerosol precursor material to the fluid-transfer article 34.

In Figures 5 to 7, the aerosol carrier is shown as comprising the fluid-transfer article 34 located within housing 32. The material forming the fluid transfer article 34 comprises a porous structure, where pore diameter size varies between one end of the fluid-transfer article 34 and another end of the fluid-transfer article. In the illustrative examples of Figs. 5 to 7, the pore diameter size gradually decreases from a first end remote from heater 24 (the upper end as shown in the figure) to a second end proximal heater 24 (the lower end as shown in the figure). Although the figure illustrates the pore diameter size changing in a step-wise manner from the first to the second end (i.e. a first region with pores having a diameter of a first size, a second region with pores having a diameter of a second, smaller size, and a third region with pores having a diameter of a third, yet smaller size), the change in pore size from the first end to the second end may be gradual rather than step-wise. This configuration of pores having a decreasing diameter size from the first end and second end can provide a wicking effect, which can serve to draw fluid from the first end to the second end of the fluid-transfer article 34.

The fluid-transfer article 34 comprises a first region 34a for holding an aerosol precursor. In one or more arrangements, the first region 34a of the fluid-transfer article 34 comprises a reservoir for holding the aerosol precursor. The first region 34a can be the sole reservoir of the aerosol carrier 14, or it can be arranged in fluid communication with a separate reservoir, where aerosol precursor is stored for supply to the first region 34a.

The fluid-transfer article 34 also comprises a second region 34b. Aerosol precursor is drawn from the first region 34a to the second region 34b by the wicking effect of the substrate material forming the fluid transfer article. Thus, the first region 34a is configured to transfer the aerosol precursor to the second region 34b of the article 34.

In Figures 5 and 6, there is a valve 34c between the first region of 34a and the second region 34b of the fluid-transfer article 34. The valve 34c regulates fluid transfer between the first and second regions 34a and 34b to control transfer of the aerosol precursor to the second region 34b. The valve 34c is heat activatable and responds to a rise in temperature to open to allow aerosol precursor to pass therethrough.

In particular, when the fluid-transfer article is not heated (e.g. when the heater 24 is at room temperature) the valve 34c is closed. When the heater 24 is activated, the heat that is generated by the heater 24 is transferred to the valve 34c, which activates to open one or more fluid flow-paths from the first region 34a to the second region 34b. Aerosol precursor may then pass to the second region 34b.

For example, the valve 34c may be made of a material with a plurality of small holes therethrough, with the material of the valve expanding when heated to dilate the holes to allow aerosol precursor to pass through the holes. If the holes dilate from 0.1mm to 1mm, when the temperature of the heater 24 rises from room (ambient) temperature to its operating temperature, the holes should enlarge sufficiently from a state in which they substantially block passage of aerosol precursor to a state in which they allow relatively free flow of aerosol precursor therethrough. There would need to be enough holes in the material to allow sufficient aerosol precursor to pass to the second region 34b when the heater 24 is active to generate sufficient vapour for the user. The material of such a valve would normally need to be chosen to resist degradation due to the elevated temperatures to which it will be exposed (typically 200 - 250°C).

Another possibility is to provide a plurality of valves in an array between the first and second regions 34a, 34b. Yet another possibility may be for there to be one, or a plurality, of electro-mechanical valves whose action is controlled by a heat sensor responsive to the heat generated by the heater 24.Other valve arrangements are also possible.

The first region 34a of the fluid transfer article, need not be of porous polymer material as described above. It may instead be a simple hollow reservoir filled with liquid aerosol precursor. The liquid aerosol precursor will then pass directly from the first region 34a to the valve 34c.

At the second end of fluid-transfer article 34, the surface of the second region 34b defines an activation surface 38, which is disposed opposite a surface for conveying heat to the activation surface 38. In the illustrative examples of Figures. 5 and 6, the opposing surface for conveying heat to the activation surface 38 comprises a conduction element 36. The conduction element 36 is located for thermal interaction with heater 24 and is arranged to transfer heat from heater 24 to the activation surface 38. As noted above, however, the conduction element 36 may be absent in some arrangements, in which case the activation surface 38 is disposed to receive heat directly from the heater 24.

The conduction element 36, if present, may comprise a thin film of thermally conductive material, such as, for example, a metal foil (for example, aluminium, brass, copper, gold, steel, silver, or an alloy comprising anyone of the foregoing together with thermally conductive plastics and/or ceramics).

The activation surface 38 may be discontinuous such that at least one channel 40 is formed between the activation surface 38 and the conduction element 36 (or the heater 24 in the case of arrangements in which the conduction element 36 is absent). In some arrangements, the discontinuities may be such that the activation surface 38 is undulating.

In the illustrative examples of Figures 5 and 6, the activation surface 38 comprises a plurality of grooves or valleys therein to form an undulating surface, the grooves or valleys being disposed in a parallel arrangement across the activation surface 38. Thus, there are a plurality of channels 40 between the activation surface 38 and the conduction element 36.

In the illustrative example of Figure 5, the grooves or valleys in the activation surface 38 provide alternating peaks and troughs that give rise to a "saw-tooth" type profile. In one or more optional arrangements, the activation surface may comprise a "castellated" type profile (i.e. a "square wave" typeprofile), for example, such as illustrated in the example of Figure. 6. In one or more optional arrangements, the activation surface may comprise a "sinusoidal" type profile. The profile may comprise a mixture of two or more of the above profiles given as illustrative examples.

In the illustrative examples of Figures 5 and 6, the first region 34a of the fluid-transfer article 34 is located at an "upstream" end of the fluid-transfer article 34 and the second region 34b is located at a downstream" end of the fluid-transfer article 34. That is, aerosol precursor is wicked, or is drawn, from the "upstream" end of the fluid-transfer article 34 to the "downstream" end of the fluid-transfer article 34 (as denoted by arrow A in Figure 5).

The aerosol precursor is configured to release an aerosol and/or vapour upon heating. Thus, when the activation surface 38 receives heat conveyed from heater 24, the aerosol precursor held at the activation surface 38 is heated. The aerosol precursor, which is captively held in material of the fluid-transfer article at the activation surface 38 is released into an air stream flowing through the channels 40 between the conduction element 36 and activation surface 38 (or between the heater 24 and the activation surface 38) as an aerosol and/or vapour.

The shape and/or configuration of the activation surface 38 and the associated shape(s) and/or configuration(s) of the one or more channels 40 formed between the activation surface 38 and conduction element 36 (or between the activation surface 38 and heater 24) permit air to flow across the activation surface 38 (through the one or more channels 40) and also increase the surface area of the activation surface 38 of the fluid-transfer article 34 that is available for contact with a flow of air across the activation surface 38.

In the embodiments of Figures 5 and 6, the valve 34c is between the first region 34a and the second region 34b of the fluid-transfer article 34. Figure 7 illustrates another embodiment in which the valve 34c forms the second region of the fluid-transfer article. Thus, in this embodiment, the valve is the closest part of the fluid-transfer article 34 to the heater 24 and the activation surface 38 is formed by a surface of the valve 34c. When the valve 34c is closed, aerosol precursor cannot pass through the second region. When the heater is activated, thereby opening the valve 34c, as the valve is activated by heat, aerosol precursor may then reach the activation surface 38.

Note that in Figure 7, the first region of the fluid-transfer article is formed of two parts 34a and 34b of different pore sizes. It would also be possible to have an arrangement in which the first region has only one part of a uniform pore size.

In the embodiment of Figure 7, the valve 34c may be made separable from the first region of the fluid-transfer article, i.e. there is no fixing of the valve 34c to the second part 34b. Since the aerosol precursor will be depleted, as the user uses the apparatus, it may be desirable to replace the first region formed by parts 34a, 34b. The separability of the valve 34c means that the valve does not need to be replaced when replacing the first region.

In the embodiment of Figure 7, there are no channels 40. However, there is an opening 37a in the housing 32, the opening 37a is in communication with the air-intake 20 of the apparatus. A further opening 37b communicates with a duct 37c within the housing 32, which duct 37c communicates with the second end 18. There is thus a fluid-flow path for air (hereafter referred to as an air-flow pathway) between openings 37a and 37b, linking the apertures 20 from the second end 18 of the aerosol carrier. That air-flow pathway extends between the activation surface 38 and the conduction element 36 of the heater 24. Thus, when the heater 24 is activated, heat is transferred from the heater 24 to the conduction element 36, and from there to the valve 34c, which opens as it is heat-activatable, to allow aerosol precursor to pass therethrough, to the activation surface 38. At activation surface 38, aerosol precursor is heated to form vapour and/or aerosol. When the user sucks or inhales, air flows between the openings 37a and 37b, and flows along the air-flow pathway between the activation surface 38 and the conduction element 36, and vapour and/or a mixture of aerosol and vapour is mixed with that air and then passes through the passageway 40 to the second end 18.

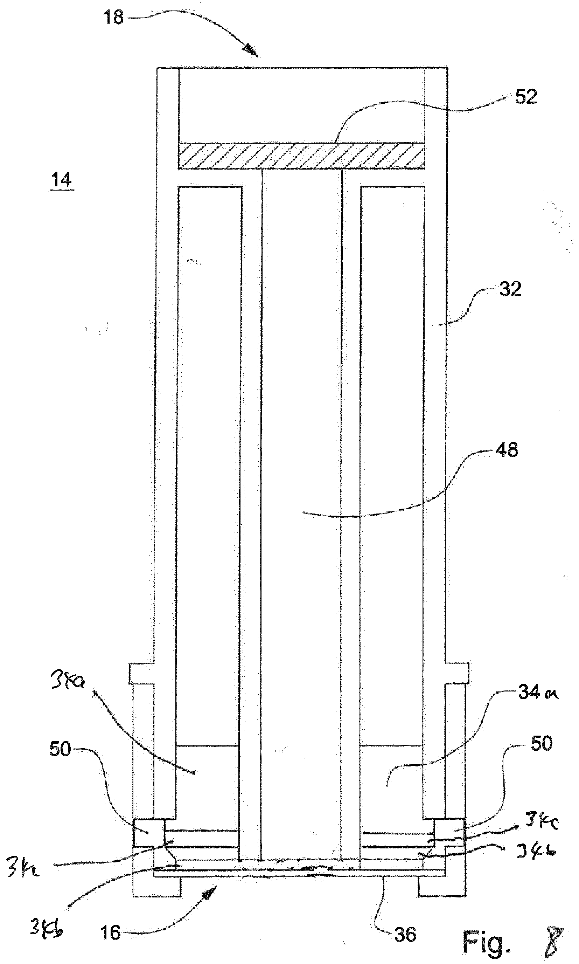

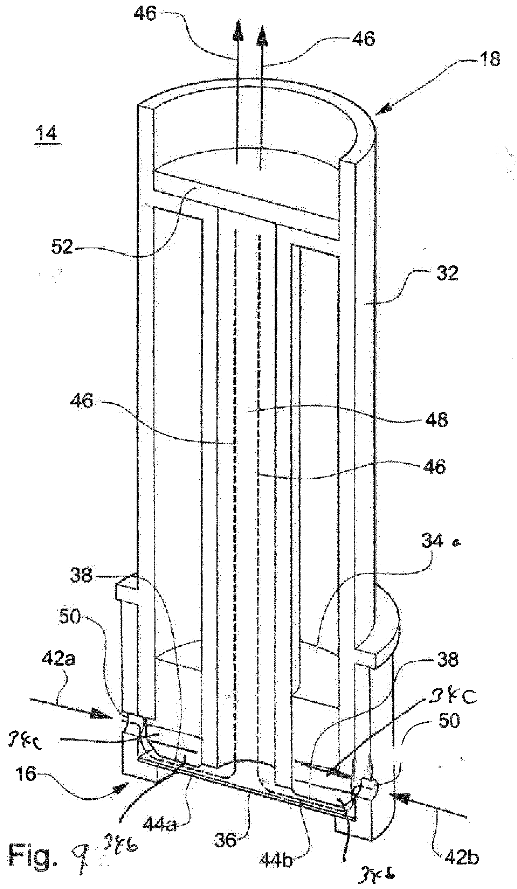

In the arrangements shown in Figures 5 to 7, the apertures 37a and 37b are on opposite sides of the housing 32. Figures 8 and 9 show an alternative configuration, in which the fluid-transfer article is annular and the valve 34c is then also annular. The arrangement of Figures 8 and 9 corresponds to the arrangement shown in Figures 5 and 6, in that there is a valve 34c between the first region 34a and second region 34b of the fluid-transfer article. Thus, Figures 8 and 9 illustrate an aerosol carrier 14 according to one or more possible arrangements in more detail. Figure 8 is a cross-section side view illustration of the aerosol carrier 14 and Figure 9 is a perspective cross-section side view illustration of the aerosol carrier 14 of Figure 8.

As can be seen from Figures 8 and 9, the aerosol carrier 14 is generally tubular in form. The aerosol carrier 14 comprises housing 32, which defines the external walls of the aerosol carrier 14 and which defines therein a chamber in which are disposed the fluid-transfer article 34 (adjacent the first end 16 of the aerosol carrier 14) and internal walls defining the fluid communication pathway 48. Fluid communication pathway 48 defines a fluid pathway for an outgoing air stream from the channels 40 to the second end 18 of the aerosol carrier 14. In the examples illustrated in Figures 11 and 12, the fluid-transfer article 34 is an annular shaped element located around the fluid communication pathway 48.

In walls of the housing 32, there are provided inlet apertures 50 to provide a fluid communication pathway for an incoming air stream to reach the fluid-transfer article 34, and particularly the one or more channels 40 defined between the activation surface of the fluid-transfer article 34 and the conduction element 36 (or between the activation surface and the 15 heater).

In the illustrated example of Figures 8 and 9, the aerosol carrier 14 further comprises a filter element 52. The filter element 52 is located across the fluid communication pathway 48 such that an outgoing air stream passing through the fluid communication pathway 48 passes through the filter element 52.

With reference to Figure 9, when the heater 24 is activated to open the valve 34c, and a user sucks on a mouthpiece of the apparatus (or on the second end 18 of the aerosol carrier 14, if configured as a mouthpiece), air is drawn into the carrier through inlet apertures 50 extending through walls in the housing 32 of the aerosol carrier 14. An incoming air stream 42a from a first side of the aerosol carrier 14 is directed to a first side of the activation surface 38 of the fluid-transfer article 34 (e.g. via a gas communication pathway within the housing of the carrier). An incoming air stream 42b from a second side of the aerosol carrier 14 is directed to a second side of the activation surface 38 of the fluid-transfer article 34 (e.g. via a gas communication pathway within the housing of the carrier). When the incoming air stream 42a from the first side of the aerosol carrier 14 reaches the first side of the activation surface 38, the incoming air stream 42a from the first side of the aerosol carrier 14 flows across the activation surface 38. Likewise, when the incoming air stream 42b from the second side of the aerosol carrier 14 reaches the second side of the activation surface 38, the incoming air stream 42b from the second side of the aerosol carrier 14 flows across the activation surface 38. The air streams from each side are denoted by dashed lines 44a and 44b in Figure 9. As air streams 44a and 44b flow, since the heater 35 is activated and the valve 34c is open, aerosol precursor can reach the activation surface 38. Then aerosol precursor in the activation surface 38, across which the air streams 44a and 44b flow, is released from the activation surface 38 by heat conveyed to the activation surface from the heater 24. Aerosol precursor released from the activation surface 38 is entrained in air streams 44a and 44b.

In use, the heater 24 of the apparatus 12 conveys heat to the activation surface 38 of the fluid-transfer article 34 to raise a temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article 34. As the air streams 44a and 44b continue their passages, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they pass through filter element 52 and exit outlet fluid communication pathway 48, either as a single outgoing air stream, or as separate outgoing air streams 46 (as shown). The outgoing air streams 46 are directed to an outlet, from where it can be inhaled by the user directly (if the second end 18 of the aerosol capsule 14 is configured as a mouthpiece), or via a mouthpiece. The outgoing air streams 46 entrained with aerosol precursor are directed to the outlet (e.g. via a gas communication pathway within the housing of the carrier).

In Figures 8 and 9 the heat activatable valve 34c is between the first and second regions 34a and 34b, as in the arrangement so Figures 5 and 6. Alternatively, the valve 34c may form the second region, as in the embodiment of Figure 7. In either case, the valve 34c controls the movement of the aerosol precursor to the activation surface 38.

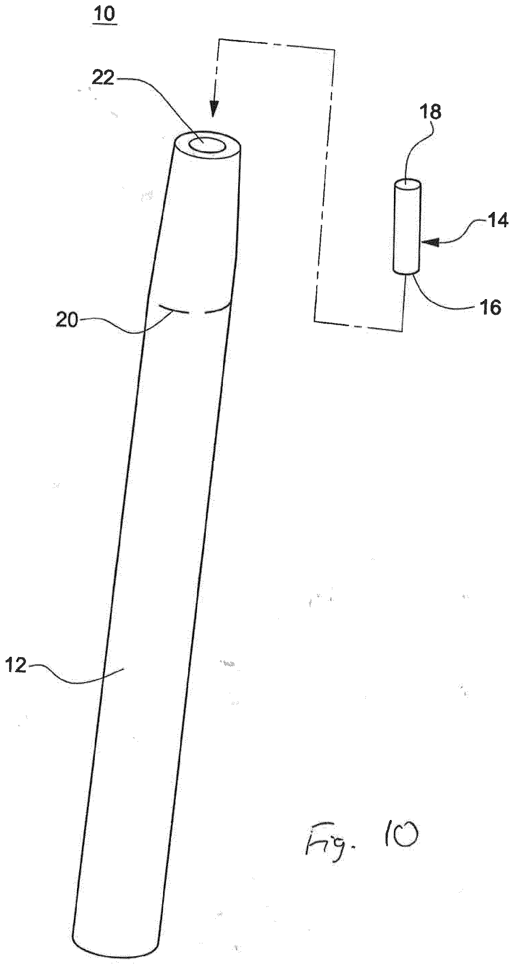

Figure 10 is an exploded perspective view illustration of a kit-of-parts for assembling an aerosol delivery system 10

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A fluid-transfer article comprising a first region for holding an aerosol precursor and for transferring said aerosol precursor to an activation surface of a second region of said article, said activation surface being disposed at an end of said article configured for thermal interaction with a heating surface of a heater of an aerosol-generation apparatus; the article further including at least one heat activatable valve for controlling the transfer of said aerosol precursor from said first region to said activation surface.

2. A fluid-transfer article according to claim 1, wherein said at least one heat activatable valve is between said first region and said second region.

3. A fluid-transfer article according to claim 2, wherein said second region is formed from a polymeric wicking material.

4. A fluid-transfer article according to claim 2 or claim 3, wherein said second region comprises at least one discontinuity in said activation surface to form a corresponding at least one channel between said activation surface and said heating surface, the or each said channel being configured for providing an air-flow pathway across said activation surface.

5. A fluid-transfer article according to claim 4, wherein said activation surface includes at least one angled surface portion and being configured said that, when said fluid transfer article is arranged with respect to said heating surface for thermal interaction therebetween, the or each angled surface portion forms an acute intersection angle with said heating surface, the or each said channel being at least partially defined by a said angled surface portion in the form of a wall of said channel.

6. A fluid-transfer article according to claim 5, wherein the or each said channel is a least partially defined by a pair of said angled surface portions, said pair of angled surface portions opposing one another across said channel to form opposite walls of said channel.

7. A fluid-transfer article according to claim 4, wherein said activation surface includes at least one arcuate surface portion and being configured such that, when the fluid transfer article is arranged with respect to said heating surface for thermal interaction therebetween, the or each arcuate surface portion opposes said heating surface and is concave towards said heating surface, said arcuate surface portion defining at least part of an internal surface of said channel.

8. A fluid-transfer article according to claim 1, wherein said at least one heat activatable valve forms said second region and said activation surface is a surface of said at least one heat activatable valve.

9. A fluid-transfer article according to claim 8, wherein said first and second regions of said fluid-transfer article are separable.

10. A fluid-transfer article according to any one of the preceding claims, wherein said first region is formed from a polymeric wicking material.

11. A fluid-transfer article according to claim 3 or claim 10, wherein said polymeric wicking material is porous.

12. An aerosol-generation apparatus comprising a heater having a heating surface and a fluid-transfer article according to any one of the preceding claims.

13. An aerosol-generation apparatus according to claim 12 as dependent on claim 8, where said activation surface is spaced from said heating surface of said heater to form an air-flow pathway therebetween.

14. An aerosol-generation apparatus according to claim 12 or claim 13, wherein said heater includes a heat conduction element at said heating surface of said heater facing said activation surface.

15. An aerosol delivery system comprising an aerosol-generation apparatus according to claim 13 or claim 14 as dependent on claim 13 and a carrier, the carrier comprising a housing containing said heater and said fluid-transfer article, said housing having an inlet and an outlet in communication with said air-flow pathway.
